(19) Europäisches Patentamt
European Patent Office
Office européen des brevets

(11) Publication number: **0 381 478**
**A1**

(12) **EUROPEAN PATENT APPLICATION**

(21) Application number: **90301016.3**

(22) Date of filing: **31.01.90**

(51) Int. Cl.[5]: **C12P 17/06, C07D 309/30, C12P 7/62, C12N 1/20**

The applicant has filed a statement in accordance with Rule 28 (4) EPC (issue of a sample only to an expert). Accession number(s) of the deposit(s): ATCC 53848 and MA 6474.

(30) Priority: **01.02.89 US 304636**

(43) Date of publication of application: **08.08.90 Bulletin 90/32**

(84) Designated Contracting States: **CH DE FR GB IT LI NL**

(71) Applicant: **MERCK & CO. INC.**
**126, East Lincoln Avenue P.O. Box 2000**
**Rahway New Jersey 07065-0900(US)**

(72) Inventor: **Ferris, Michael J.**
**1402 Brown Place, Rahway**
**New Jersey 07065(US)**
Inventor: **Hirsch, Charles F.**
**2 Davis Close**
**Somerville, New Jersey 08876(US)**
Inventor: **Houck, David R.**
**10 Los Arboles Drive**
**Los Alamos, New Mexico 87544(US)**

(74) Representative: **Hesketh, Alan, Dr. et al**
**European Patent Department Merck & Co., Inc. Terlings Park Eastwick Road**
**Harlow Essex, CM20 2QR(GB)**

(54) **Process for the preparationn of 6-alpha-hydroxymethyl lovastatin derivatives.**

(57) A microbiological process is described for the preparation of 6-desmethyl-6-α-hydroxymethyl lovastatin derivatives and 8-acyl analogs thereof.

**EP 0 381 478 A1**

# PROCESS FOR THE PREPARATION OF 6-α-HYDROXYMETHYL LOVASTATIN DERIVATIVES

## BACKGROUND OF THE INVENTION

Hypercholesterolemia is known to be one of the prime risk factors for atherosclerosis and coronary heart disease, the leading cause of death and disability in western countries. The bile acid sequestrants seem to be moderately effective as antihypercholesterolemic agents but they must be consumed in large quantities, i.e., several grams at a time, and then are not very palatable.

MEVACOR® (lovastatin), now commercially available, is one of a group of very active antihypercholesterolemic agents that function by limiting cholesterol biosynthesis by inhibiting the enzyme, HMG-CoA reductase. In addition to the natural fermentation products, mevastatin and lovastatin, there are a variety of semi-synthetic and totally synthetic analogs thereof. For example, simvastatin wherein the 8-acyl moiety is 2,2-dimethylbutyryl is an even more potent HMG-CoA reductase inhibitor than lovastatin.

The naturally occurring compounds and their semi-synthetic analogs have the following general structural formulae:

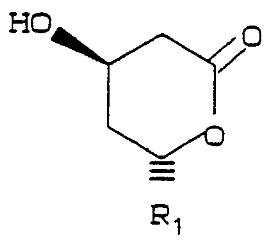

or

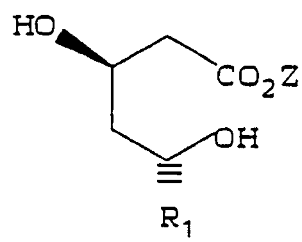

wherein:
Z is hydrogen, $C_{1-5}$ alkyl or $C_{1-5}$ alkyl substituted with a member of the group consisting of phenyl, dimethylamino, or acetylamino; and
$R_1$ is:

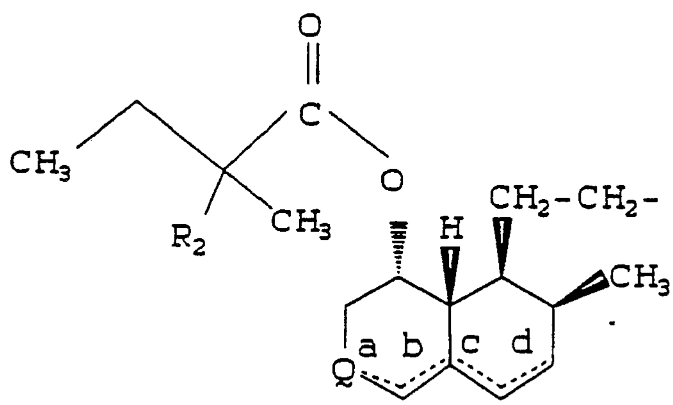

wherein Q is

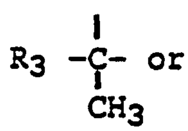 or 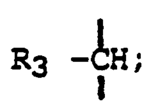;

$R_3$ is H or OH; and
$R_2$ is hydrogen or methyl; and a, b, c, and d represent optional double bonds, especially where b and d represent double bonds or a, b, c, and d are all single bonds, provided that when a is a double bond, Q is

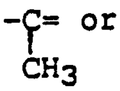 or 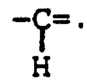

U.S. Patent 4,517,373 discloses semi-synthetic hydroxy containing compounds represented by the above general formula wherein $R_1$ is

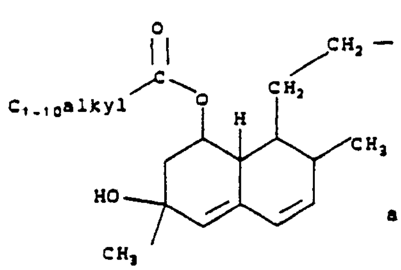

and

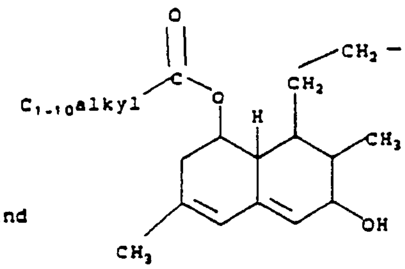

U.S. Patent 4,537,859 and U.S. Patent 4,448,979 also disclose semi-synthetic hydroxy-containing compounds represented by the above general formula wherein $R_1$ is

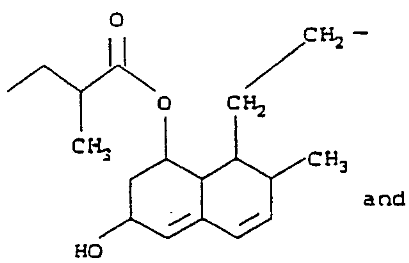

and

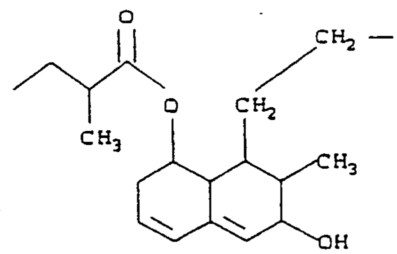

These compounds are prepared by the action of certain microorganisms on the corresponding non-hydroxylated substrates. One such organism described in U.S. 4,537,859 is of the genus Nocardia.

Copending U.S. patent application 048,136 filed May 15, 1987, corresponding to EP 251625 discloses HMG-CoA reductase inhibitors which include 6-desmethyl-6-hydroxymethyl and 6-desmethyl-6-carboxysimvastatin analogs. These compounds were formed by bioconversion of the sodium salt of simvastatin using strains of the microorganism Nocardia autotrophica (MA 6180 and MA 6181). However, the bioconversions yielded predominantly, the isomer in which the 6-moiety had the beta configuration.

Copending U.S. patent applications S.N. 161, 530 and 161,579 corresponding to EP 331239 and EP 331238, describe synthetic chemical methodology for the synthesis of 6-α-hydroxymethyl analogs of lovastatin and analogs thereof at the acyl side chain.

Copending U.S. patent application S.N. 181,877 and S.N. 181,878 corresponding to EP 337548 disclose a process and microorganism for the preparation of 6-desmethyl-6-β-hydroxymethyl- and 6-desmethyl-6-β-carboxylovastatin derivatives and 8-acyl analogs therof.

## DETAILED DESCRIPTION OF THE INVENTION

The instant invention is a novel process for the preparation of 6-desmethyl-6-α-hydroxymethyl derivatives of lovastatin and analogs thereof (I) employing an unidentified Actinomycete (MA6474).

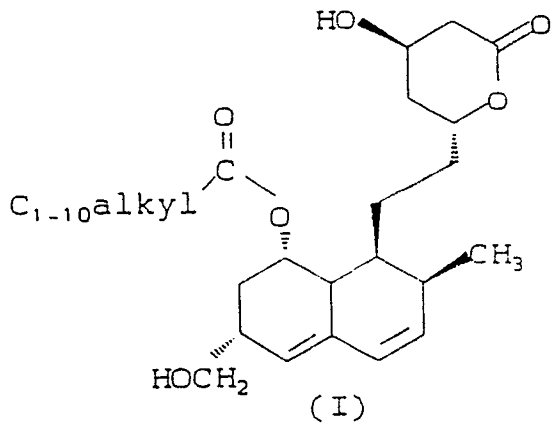

(I)

The process involves the bioconversion of substrate (II) with a culture containing an unidentified Actinomycete (MA6474).

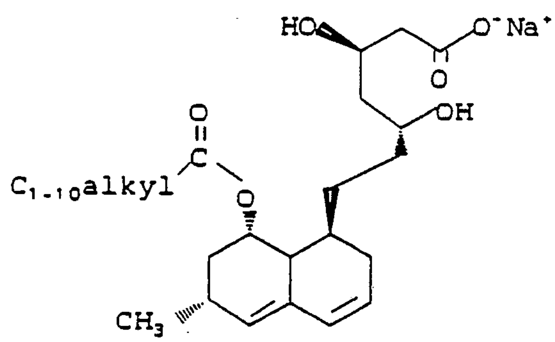

(II)

The acyl moiety

$C_{1-10}$alkyl- $\overset{O}{\overset{\|}{C}}$ - can be branched or straight, preferably it is 2-methylbutyryl or 2,2-dimethylbutyryl, most preferably 2,2-dimethylbutyrl.

The characteristics of the unidentified Actinomycete (MA6474) are described below:

Cultural Characteristics

Oatmeal Agar

Vegetative Growth: Reverse: tan. Obverse: tan, raised growth
Aerial Mycelium: Sparse, white, powdery
Soluble Pigment: None

Czapek-Dox Agar (Sucrose Nitrate Agar)

Vegetative Growth: Light yellow, matte, flat. One plate demonstrates clearing around periphery of growth
Aerial Mycelium: None
Soluble Pigment: None

Egg Albumin Agar

Vegetative Growth: Yellow, flat, matte.
Aerial Mycelium: None
Soluble Pigment: None

Glycerol Asparagine Agar

Vegetative Growth: Reverse: Creamy yellow, flat, matte. Obverse: Same
Aerial Mycelium: None
Soluble Pigment: None

Inorganic Salts-Starch Agar

Vegetative Growth: Yellow, flat matte
Aerial Mycelium: None
Soluble Pigment: None

Nutrient Tyrosine Agar
Vegetative Growth: Reverse: Tan
Aerial Mycelium: None
Soluble Pigment: None
Decomposition of tyrosine: Observed in areas of heavy growth
Skim Milk Agar
Vegetative Growth: Yellow-tan, flat, matte Aerial Mycelium: None
Soluble Pigment: Light Brown
Hydrolysis of Casein: Excellent
Yeast Extract-Malt Extract Agar
Vegetative Growth: Reverse: tan-brown. Obverse: tan-brown, raised, rugose
Aerial Mycelium: Greyish-white powdery
Soluble pigment: None
Nutrient Agar
Vegetative Growth: Yellow-tan, raised, rugose, matte
Aerial Mycelium: None
Soluble Pigment: None
Nutrient Starch Agar
Vegetative Growth: Yellow-tan, raised, rugose, matte
Aerial Mycelium: None
Soluble Pigment: None
Hydrolysis of Starch: Good
Tomato Paste-Oatmeal Agar
Vegetative Growth: One plate demonstrates rugose, "heaped up" mounds of mustard-yellow vegetative growth. Second plate displays confluent brown-yellow rugose growth and also isolated mustard-yellow, rugose, "heaped up" colonies
Aerial Mycelium: Sparse white aerial mycelium on second plate cited above
Soluble Pigment: None
Gelatin Stabs
Vegetative Growth: None
Aerial Mycelium: None
Soluble Pigment: None
Peptone-Iron-Yeast Extract Agar
Vegetative Growth: Orange-brown raised rugose, erose edges
Aerial Mycelium: None
Soluble Pigment: None
Melanin: Negative
$H_2S$: Negative
Czapek-Dox Agar Slants
Vegetative Growth: Colorless
Aerial Mycelium: None
Soluble Pigment: None
Morphological Characteristics
Branched filaments, filament diameter approximately 0.76 microns. Spores produced in short chains 2-10 spores coiled and appear as spherical to ovoid form (0.76 x 1.0 microns). Spores appear to be borne in sessile sporangia, 4-10 microns diameter.
Physiological and Biochemical Characteristics

Oxygen Requirements (Stab Culture in Yeast Extract-Dextrose + Salts Agar) Aerobic

## Carbon Utilization

Pridham-Gottlieb Basal Medium + 1% carbon source: graded according to standards in "Methods for Characterization of Streptomyces Species" International Journal of Systematic Bacteriology, Vol. 16, No. 3, July 1966, pps. 313-340.

| | |
|---|---|
| NS (No Carbon Source) | light growth |
| alpha-D-Glucose (Positive Control) | excellent growth |
| D-Arabinose | + |
| L-Arabinose | + |
| D-Fructose | ++ |
| L-Glucose | - |
| Inositol | - |
| alpha-D-Lactose | - |
| beta-D-Lactose | - |
| D-Maltose | ++ |
| D-Mannitol | + |
| D-Mannose | ++ |
| L-Mannose | - |
| D-Raffinose | - |
| L-Rhamnose | + |
| Sucrose | - |
| D-Xylose | ++ |
| L-Xylose | - |

All readings taken after three weeks at 28°C unless noted otherwise. pH of all media approximately neutral (6.8-7.2).

A deposit of the unidentified Actinomycete (MA6474) has been made (November 11, 1988) under the Budapest Treaty. The deposited culture designated ATCC 53828 is available in the permanent culture collection of the American Type Culture Collection at 12301 Parklawn Drive, Rockville, MD 20852.

The compounds (I) are prepared in the instant process from the sodium salt of simvastatin, lovastatin or an analog having a 6-methyl group by one of the following methods:

(a) adding the substrate to a growing culture of the unidentified Actinomycete for a suitable incubation period followed by isolation, and derivatization if desired;

(b) collecting a culture of the bioconverting microorganism and contacting the collected cells with the substrate.

Cultivation of the bioconverting microorganism can be carried out by conventional means in a conventional culture medium containing nutrients well known for use with such microorganisms. Thus, as is well known, such culture media contain sources of assimilable carbon and of assimilable nitrogen and often inorganic salts. Examples of sources of assimilable carbon include glucose, sucrose, starch, glycerin, millet jelly, molasses and soybean oil.

Examples of sources of assimilable nitrogen include soybean solids (including soybean meal and soybean flour), wheat germ, meat extracts, peptone, corn steep liquor, dried yeast and ammonium salts,

such as ammonium sulphate. If required, inorganic salts, such as sodium chloride, potassium chloride, calcium carbonate or phosphates, may also be included. Also, if desired other additives capable of promoting the production of hydroxylation enzymes may be employed in appropriate combinations. The particular cultivation technique is not critical to the process of the invention and any techniques conventionally used for the cultivation of microorganisms may be employed with the present invention. In general, of course, the techniques employed will be chosen having regard to industrial efficiency. Thus, liquid culture is generally preferred and the deep culture method is most convenient from the industrial point of view.

Cultivation will normally be carried out under aerobic conditions and at a temperature within the range from 20° to 37° C., more preferably from 26° to 28° C.

Method (a) is carried out by adding the substrate to the culture medium in the course of cultivation. The precise point during the cultivation at which the starting compound is added will vary depending upon the cultivation equipment, composition of the medium, temperature of the culture medium and other factors, but it is preferably at the time when the hydroxylation capacity of the microorganism begins to increase and this is usually 1 or 2 days after beginning cultivation of the microorganism. The amount of the substrate added is preferably from 0.01 to 5.0% by weight of the medium, more preferably from 0.05 to 0.5%, e.g., from 0.05 to 0.1% by weight.

After addition of the substrate, cultivation is continued aerobically, normally at a temperature within the ranges proposed above. Cultivation is normally continued for a period of from 1 to 2 days after addition of the substrate.

In method (b), cultivation of the microorganism is first carried out under conditions such as to achieve its maximum hydroxylation capacity; this capacity usually reaches a maximum between 4 and 5 days after beginning the cultivation, although this period is variable, depending upon the nature and temperature of the medium, the species of microorganism and other factors. The hydroxylation capacity of the culture can be monitored by taking samples of the culture at suitable intervals, determining the hydroxylation capacity of the samples by contacting them with a substrate under standard conditions and determining the quantity of product obtained and plotting this capacity against time as a graph. When the hydroxylation capacity has reached its maximum point, cultivation is stopped and the microbial cells are collected. This may be achieved by subjecting the culture to centrifugal separation, filtration or similar known separation methods. The whole cells of the cultivating microorganism thus collected, preferably, are then washed with a suitable washing liquid, such as physiological saline or an appropriate buffer solution.

Contact of the collected cells of the undentified Actinomycete with the substrate is generally effected in an aqueous medium, for example in a phosphate buffer solution at a pH value of from 5 to 9. The reaction temperature is preferably within the range from 20° to 45° C., more preferably from 25° to 30° C. The concentration of the substrate in the reaction medium is preferably within the range from 0.01 to 5.0% by weight. The time allowed for the reaction is preferably from 1 to 5 days, although this may vary depending upon the concentration of the substrate in the reaction mixture, the reaction temperature, the hydroxylation capacity of the microorganism (which may, of course, vary from species to species and will also, as explained above, depend upon the cultivation time) and other factors.

The microorganism useful in the novel process of this invention is an unidentified Actinomycete - (MA6474). A sample of the culture designated ATCC 53828 is available in the permanent culture collection of the American Type Culture Collection at 12301 Parklawn Drive, Rockville, MD 20852.

After completion of the conversion reaction by any of the above methods, the desired compound can be directly isolated, separated or purified by conventional means. For example, separation and purification can be effected by filtering the reaction mixture, extracting the resulting filtrate with a water-immiscible organic solvent (such as ethyl acetate), distilling the solvent from the extract, subjecting the resulting crude compound to column chromatography, (for example on silica gel or alumina) and eluting the column with an appropriate eluent, especially in an HPLC apparatus.

The following examples illustrate the preparation of these compounds and, as such, are not to be construed as limiting the invention set forth in the claims appended hereto.

The composition of media employed in the following examples are listed below.

| KE MEDIUM | |
|---|---|
| Component | (g/L) |
| Glucose | 1.0 |
| Soluble starch | 10.0 |
| Beef extract | 3.0 |
| Ardamine | 5.0 |
| NZ Amine E | 5.0 |
| $MgSO_4 \cdot 7H_2O$ | 0.05 |
| Phosphate buffer (see below) | 2.0 ml |
| $CaCO_3$ | 0.5 g |
| Distilled $H_2O$ | 1000 ml |
| pH = 7.0 - 7.2 (adjust with NaOH) | |
| Phosphate buffer | |
| $KH_2PO_4$ | 91.0 g |
| $Na_2HPO_4$ | 95.0 g |
| Distilled $H_2O$ | 1000 ml |
| pH = 7.0 | |

| BAM-2 MEDIUM | |
|---|---|
| Component | (g/L) |
| Yeast extract | 1.0 |
| Beef extract | 1.0 |
| Casein Hydrolysate | 2.0 |
| Glucose | 10.0 |
| pH = 7.0 (adjust with tetramethylammonium hydroxide) | |

## EXAMPLE 1

Preparation of 6(R)-[2-[8(S)-(2,2-dimethylbutyryloxy)-2(S)-methyl-6(R)-hydroxymethyl-1,2,6,7,8,8a(R)-hexahydronaphthyl-1(S)ethyl]-4(R)-3,4,5,6-tetrahydro-2H-pyran-2-one

### A. Culture conditions and Bioconversion

An agar plus inoculum was asceptically transfered from a BAM-2 plate, which contained visible growth of MA6474, to a 250 ml baffled flask containing 50 ml of BAM-2 broth. The culture was incubated at 28° C on a rotary shaker at 220 rpm for 6 days, sodium 7-[1,2,6,7,8,8a(R)-hexahydro-2(S)-methyl-6(R)-methyl-8-(S)-(2,2-dimethylbutyryloxy)-1(S)-naphthyl]-3(R),5(R)-dihydroxyheptanoate (sodium salt) was then added to a final concentration of 100 µg/ml and incubation was continued for 48 hours, at 27° C.

### B. Isolation

The whole broth from 20 flasks, prepared as above, was pooled, clarified (3000 rpm, 10 minutes) and adjusted to pH 6.0 (20% $H_3PO_4$). At a flow rate of 4 ml/min., 800 ml of clarified broth was loaded onto a SP-207 column (1.5 x 25 cm, Mitsubishi Chem.) the resin of which had previously been washed with $CH_3CN$ and thoroughly equilibrated with 10 mM, $NH_4H_2PO_4$, PH 6.4 (no traces of $CH_3CN$). The resulting broth was then eluted with the following solvent mixtures in the order: water (250 ml), 10% aqueous $CH_3CN$/300 ml), 15% aqueous $CH_3CN$ (250 ml), and 25% aqueous $CH_3CN$ (250 ml). The last fraction was adjusted to pH = 4.0 with $H_3PO_4$ and the metabolites extracted into two volumes of $CH_2Cl_2$ (2 x 250 ml). After the solvent was evaporated (in vacuo, 40°), the resulting oil was dissolved in 100 ml benzene, which contained 5 ml $CF_3COOH$. Lactonization was accomplished by heating this mixture for 15 minutes at 40° C. The benzene was evaporated in vacuo, and the sample dissolved in a minimum volume of $CH_3CN$ (0.6 ml). Semi-preparative HPLC employed a column of Zorbax $C_{18}$ (1 x 25 cm) which was eluted with a linear gradient of 34 to 46% aqueous $CH_3CN$ at 2.5 ml/min, with detection at 255 nm. The titled compound was found to have a retention time of 17.8 minutes whereas the epimeric 6-(S)-hydroxymethyl analog had a retention time of 19.0 minutes. Fractions containing the titled compound were extracted with 4 ml benzene. Evaporation of the solvent yielded the titled compound.

## Claims

1. A process for the preparation of a compound represented by the formulae (I)

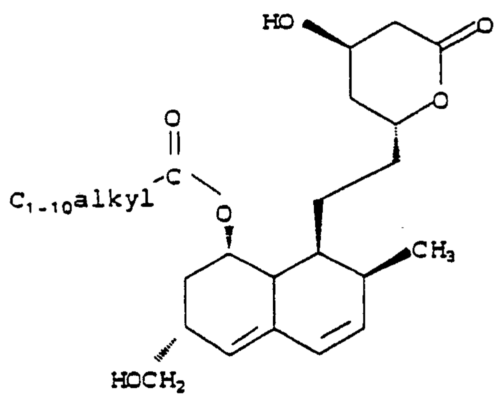

(I)

which comprises culturing a microorganism MA6474 (ATCC 53828) in a nutrient medium containing assimilable sources of nitrogen and carbon and the sodium salt (II):

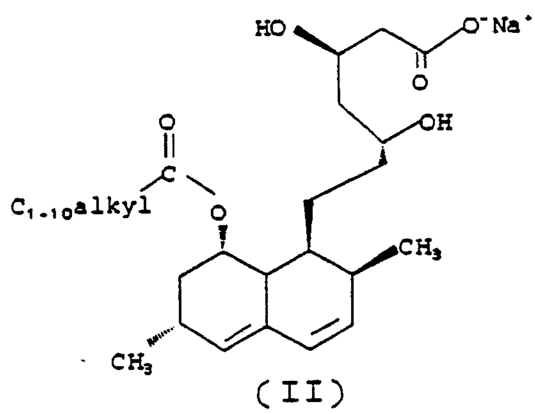

(II)

under aerobic conditions until a substantial amount of the compound is produced and isolating the compound so produced.

2. A process of Claim 1 in which the culturing of the microorganism occurs at a pH of about 7.

3. A process of Claim 2 in which $C_{1-10}\text{alkyl-}\underset{\underset{O}{\|}}{C}\text{-}$ is 2-methylbutyryl or 2,2-dimethylbutyryl.

4. A process of Claim 3 in which $C_{1-10}\text{alkyl-}\underset{\underset{O}{\|}}{C}\text{-}$ is 2,2-dimethylbutyryl.

5. A process of Claim 4 in which $C_{1-10}\text{alkyl-}\underset{\underset{O}{\|}}{C}\text{-}$ is 2-methylbutyryl.

6. A biologically pure culture of MA6474 (ATCC 53828), or an active mutant thereof, said microorganism being capable, following the description of Claim 1, of producing a compound of formula (I).

7. A culture of the microorganism according to Claim 6 which is capable of producing a compound of formula (I).

European Patent Office

# EUROPEAN SEARCH REPORT

Application number

EP 90301016.3

## DOCUMENTS CONSIDERED TO BE RELEVANT

| Category | Citation of document with indication, where appropriate, of relevant passages | Relevant to claim | CLASSIFICATION OF THE APPLICATION (Int Cl 5) |
|---|---|---|---|
| D,X, P | EP - A2 - 0 337 548 (MERCK & CO.INC.) * Claims 1,3-5 * | 1,3-5 | C 12 P 17/06<br>C 07 D 309/30<br>C 12 P 7/62<br>C 12 N 1/20 |
| D,X | EP - A2 - 0 251 625 (MERCK & CO.INC.) * Claim 1 * | 1 | |
| D,X | US - A - 4 517 373 (TERAHARA et al.) * Claims; column 4 * | 1 | |
| P,X | EP - A1 - 0 325 817 (MERCK & CO.INC.) * Claim 1 * | 1 | |
| P,X | EP - A1 - 0 323 866 (MERCK & CO.INC.) * Claim 1 * | 1 | |

TECHNICAL FIELDS SEARCHED (Int Cl 5)

C 12 P
C 07 D 309/00
C 12 N

The present search report has been drawn up for all claims

| Place of search | Date of completion of the search | Examiner |
|---|---|---|
| VIENNA | 14-03-1990 | WOLF |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document